# EUROPÄISCHE PATENTSCHRIFT

(11) **EP 1 967 147 B1**
(45) Veröffentlichungstag und Bekanntmachung des Hinweises auf die Patenterteilung: **12.05.2010**
(21) Anmeldenummer: 08102331.9
(22) Anmeldetag: 06.03.2008
(51) Int. Cl.: A61B 17/34, A61B 17/435, A61D 19/04

(54) **Punktionsvorrichtung für die Entnahme einer organischen Probe**
Puncture device for retrieving an organic sample
Dispositif de ponction pour l'extraction d'un échantillon organique

(30) Priorität: 09.03.2007 AT 3792007
(43) Veröffentlichungstag der Anmeldung: 10.09.2008
(73) Patentinhaber: Steiner, Hans-Peter, 8010 Graz (AT)
(72) Erfinder: Steiner, Hans-Peter, 8010 Graz (AT)
(74) Vertreter: Babeluk, Michael

(56) Entgegenhaltungen:
- WO-A-02/35924
- DE-A1- 3 702 441

## Beschreibung

Die Erfindung betrifft eine Punktionsvorrichtung für die Entnahme einer organischen Probe, insbesondere einer Eizelle für die In-Vitro-Fertilisierung, mit einer zu einer Nadelspitze zulaufenden Punktionskanüle, deren proximales Ende mit einem Aufnahmebehälter für die organische Probe verbindbar ist, wobei das Lumen der Punktionskanüle als Saugkanal ausgebildet ist, sowie mit einer Spülleitung, welche zur Zufuhr eines Spülmittels dient und am proximalen Ende mit einem Spülmittelbehälter verbindbar ist.

Im Rahmen der Sterilitätsbehandlung mit Hilfe der klassischen In-Vitro-Fertilisierung (IVF) werden die Eizellen durch Punktion des Eibläschens (Follikel) unmittelbar vor dem zu erwartenden Eisprung gewonnen. Mittels Hormonen wird die Reifung der Eizellen unterstützt, wodurch gleichzeitig mehrere Eizellen heranreifen und die Erfolgschancen der IVF erhöht werden. Meist werden bei der Follikel-Punktion Punktionsnadeln mit einem Außendurchmesser von 1,4 mm bis 1,5 mm und einer Länge von 30 cm verwendet, wobei pro Eibläschen (Durchmesser bis zu 2,5 cm) etwa 5 ml Follikelflüssigkeit abgesaugt werden.

Zum Teil können allerdings durch die Hormonbehandlung Schwierigkeiten auftreten, so dass für Patientinnen mit dem sogenannten PCO-Syndrom (Polyzystische Ovarien) die lebensbedrohliche Komplikation eines sogenannten ovariellen Hyperstimulationssyndroms(OHSS) auftreten kann. Ein neues Verfahren, die sogenannte In-Vitro-Maturation (IVM),verhindert diese Komplikation.

Bei der IVM wird ohne vorherige Hormongabe in einem spontanen Zyklus bereits bei einer Follikelgröße von 8 mm bis 10 mm meist zwischen dem 8. und 10. Zyklustag die Follikelreifung hormonell ausgelöst, wobei nach ca. 36 bis 38 Stunden alle vorhandenen Eibläschen ab einer Größe von 3 mm bis 4 mm punktiert und unter Ultraschallkontrolle abgesaugt werden. Die Eizellen werden dann im Brutschrank 24 Stunden nachgereift und danach im Falle des Vorhandenseins eines Polkörperchens mittels der ICSI-Methode fertilisiert.

Aufgrund der geringen Follikelgröße bei der IVM sind die meisten bekannten Punktionssets zur Gewinnung der Eizelle ungeeignet.

Es ist bekannt Punktionsvorrichtungen mit einer Spülvorrichtung zu verwenden, wobei beispielsweise mit Hilfe eines Dreiwege-Ventils, welches an eine einlumige Punktionsnadel anschließt, je nach Stellung des Ventils gespült oder aspiriert werden kann. Ein Nachteil derartiger Systeme besteht darin, dass generell ein großes Totvolumen vorhanden ist, welches dem Volumen der Punktionsvorrichtung von der Kanülenspitze bis zum Dreiwege-Ventil entspricht.

Zur Minimierung des Totvolumens wurden daher auch doppellumige Punktionskanülen eingesetzt, bei welchen eine Kanüle zur Aspiration und eine zum Spülen des Follikels dient. Der Nachteil bei doppellumigen Punktionssets besteht darin, dass der Gesamtdurchmesser relativ groß ist und für die IVM nicht geeignet ist.

Aus der US 4,493,694 A ist eine chirurgische Absaugeinrichtung mit einem Saugrohr bekannt, das von einem Ultraschallgenerator mit Ultraschallenergie beaufschlagt wird. Das Saugrohr wird von eine Hülse umgeben, so dass zwischen dem Saugrohr und der Hülse ein sich nach vorne verengender Ringspalt ausbildet. In den Ringspalt, welcher am von der Saugöffnung abgewandten Ende durch eine Dichtung zum Saugrohr abgeschlossen ist, mündet die Anschlussleitung für eine Spülflüssigkeit. Um den Materialfluss des abgesaugten Materials zu verbessern, weist der ringförmige Spalt im vorderen Bereich Übertrittsöffnungen für die Spülflüssigkeit in den Saugkanal des Saugrohrs auf. Weiters ist der durch die Hülse gebildete Ringspalt an seinem der Saugöffnung zugewandten Ende offen, so dass auch an der Außenseite des Saugrohrs Spülflüssigkeit austreten kann. Die Vorrichtung gemäß US 4,493,694 A ist unter anderem deshalb nicht geeignet, als Punktionsvorrichtung für die Entnahme einer Eizelle eingesetzt zu werden, da die zu punktierende Eizelle der in das Saugrohr eingekoppelten Ultraschallenergie ausgesetzt wäre.

Aus der WO 93/07917 ein Punktionsset zur Eizellenentnahme bekannt, welches eine als Spülkanal ausgeführte, spitz zulaufende äußere Kanüle aufweist, die eine bis in die Nadelspitze geführte innere Kanüle aufnimmt, die als Saugkanal ausgebildet ist. Der Spülkanal hat einen kreisringförmigen Querschnitt und wird durch die Außenwand der inneren Kanüle und die Innenwand der äußeren Kanüle begrenzt. Nachteilig ist der relativ große Durchmesser der doppellumigen Nadel.

Aus der WO 2005/055841 ist eine Punktionsvorrichtung zur Entnahme von Eizellen bekannt, wobei unterschiedlichste Varianten für eine Beheizung der Punktionsnadel vorgestellt werden. Neben elektrischen Heizeinrichtungen in Form einer induktiven Heizung, einer Widerstandsheizung sowie in Längsrichtung der Nadel innen oder außen angeordneten Heizdrähten, wird eine doppellumige Nadel vorgeschlagen, bei welcher im ringförmigen Spalt zwischen der Innennadel und der Außennadel eine erwärmte Flüssigkeit geführt wird. Erst nach einer ausreichenden Erwärmung der Punktionsnadel erfolgt die Gewinnung der Eizelle. Im Wesentlichen gelten auch hier die bereits im Zusammenhang mit der WO 93/07917 angeführten Nachteile.

Weiters ist aus der DE 35 22 782 A1 eine doppellumige Punktionskanüle zur Follikelpunktion bekannt geworden, welche aus einem Außenrohr mit im Wesentlichen kreisförmigen Querschnitt und einem Innenrohr besteht, dessen Außenwand in einem großen Umfangsbereich an der Innenwand des Außenrohrs anliegt, wobei das Innenrohr nur in einem kleinen Bereich mit einem Umfangswinkel kleiner 90° eine Einwölbung aufweist, welche in diesem Bereich zum Außenrohr einen Abstand aufweist und den Spülkanal bildet. Der verbleibende Saugkanal im Innenrohr weist dadurch allerdings eine den freien Durchtritt der durch Punktion gewonnenen Eizellen behindernde Einwölbung auf. Weiters besteht durch diese Einwölbung an der Eintrittsöffnung im Bereich der Nadelspitze eine Kante, welche ebenfalls störend für die Aufnahme organischer Proben ist.

Aus der EP 1 158 913 B1 ist eine Anordnung zum Übertragen einer Eizelle von einem Follikel bekannt, welche ebenfalls eine doppellumige Kanüle verwendet. Die Kanüle weist eine distal spitz zulaufende Punktionskanüle auf, deren proximales Ende mit einem Aufnahmebehälter für die Eizelle verbunden ist. Der Behälter für die Aufnahme der Eizelle weist eine Verbindung zu einer Vakuumquelle auf, so dass in der Punktionskanüle eine Saugwirkung erzielt werden kann. Der Spülschlauch der Vorrichtung ist über ein spezielles Verbindungselement am proximalen Ende der Punktionsnadel ins Innere der Punktionskanüle eingekoppelt, wo ein elastisch verformbarer Spülkanal aus einem flexiblen Material vorliegt, der bis zur Nadelspitze der Punktionsnadel reicht. Der elastische Spülkanal liegt mit einem Teil seiner Außenwand an der Innenwand der Punktionsnadel an, wobei der freiliegende Wandbereich wie eine Membran elastisch verformbar ist, so dass beim Durchtritt der Eizelle der Durchmesser des Saugkanals örtlich vergrößert werden kann. Der Aufbau der Kanüle ist relativ kompliziert, da der elastisch verformbare Spülschlauch an der Innenwand der Punktionskanüle befestigt werden muss. Weiters bildet die elastisch verformbare Wand des Spülkanals an der Eintrittsöffnung der Nadel eine störende Kante für die empfindliche Eizelle, wobei auch die Druckbelastung bei der Verformung des Spülkanals für die Eizelle schädlich ist.

Aus der US 2002/0123689 A1 ist ein Führungsadapter zur Befestigung eines chirurgischen Instruments, beispielsweise einer Biopsienadel, an einem Ultraschallkopf bekannt, wobei der Adapter aus zwei miteinander verbindbaren Teilen besteht, welche nach dem Zusammenfügen eine längliche Aufnahme für das chirurgische Instrument bilden. An einem der beiden den Führungsadapter bildenden Teile ist einstückig eine Klemmvorrichtung angeordnet, welche über ein Scharnier geöffnet werden kann und zur Befestigung des Adapters am Ultraschallgerät dient. Der Führungsadapter besteht somit aus einem zweiteiligen Führungselement, nämlich aus einem länglichen Unterteil samt Klemmelement und einem im Querschnitt U-förmigen Oberteil, welche Teile nach dem Zusammenfügen das chirurgische Instrument führen.

Aufgabe der Erfindung ist es, eine Punktionsvorrichtung für die Entnahme einer organischen Probe, insbesondere einer Eizelle, derart weiter zu entwickeln, dass deren Anwendung neben der herkömmlichen IVF auch vorteilhaft für die In-Vitro-Maturation (IVM) eingesetzt werden kann, wobei ein möglichst einfach herzustellendes Punktions-Set realisiert werden soll.

Diese Aufgabe wird erfindungsgemäß dadurch gelöst, dass im Anschluss an die Nadelspitze ein einlumiger Punktionsbereich ausgebildet ist, an welchen ausgehend von einer Verbindungsstelle, in welcher die Spülleitung flüssigkeitsdicht an der Punktionskanüle befestigt ist, ein Bereich anschließt, in welchem die Punktionskanüle in der Spülleitung verläuft, so dass zwischen der Außenwand der Punktionskanüle und der Innenwand der Spülleitung ein Spülkanal ausgebildet ist, welcher an dessen distalem Ende zumindest eine Übertrittsöffnung in den Saugkanal der Punktionskanüle aufweist.

Vorteilhafte Merkmale der Erfindung sind in den abhängigen Ansprüche enthalten.

In vorteilhafter Weise kann der einlumige Punktionsbereich der Kanüle mit einem sehr kleinen Außendurchmesser im Bereich von 0,8 mm bis 1,2 mm hergestellt werden, wodurch dieser Bereich mit einer Länge von 60 mm bis 90 mm äußerst kleinvolumig gehalten werden kann. Es entsteht daher nur ein vernachlässigbares Totvolumen von ca. 30 µl. Die Länge des einlumigen Bereiches von der Nadelspitze bis zur Verbindungsstelle mit der Spülleitung beträgt je nach Anwendungsgebiet ca. 10% bis 25% der Länge des doppellumigen Bereichs der Punktionskanüle.

Ein entscheidender Vorteil ergibt sich aus den Strömungsverhältnissen im einlumigen Punktionsbereich der erfindungsgemäßen Nadel, wobei die Strömungsrichtung in diesem Bereich ohne Verwendung eines Dreiwege-Ventils sehr rasch umgekehrt werden kann. Diese Konstruktion ermöglicht es dem Arzt sogar während des Anliegens eines Aspirationsdruckes von 80 mm/Hg an der Saugleitung mittels einer Spülmittelpumpe, z.B. eine Kolbenspritze, mehrmals nacheinander den Aspirationsdruck zu überwinden und somit den Follikel pulsativ binnen weniger Sekunden mehrfach, beispielsweise 3- bis 4-mal, zu spülen. Setzt der Arzt eine elektrisch betriebene Spülmittelpumpe ein, hat er die Option, die Aspirationspumpe beim Spülen mehrmals auf Null zu stellen und so abwechseln zu saugen und zu spülen.

Mit der erfindungsgemäßen Vorrichtung ist die Follikelpunktion aufgrund der geringen Nadeldicke wesentlich weniger schmerzhaft, sodass in den meisten Fällen eine Behandlung ohne Vollnarkose möglich ist

Erfindungsgemäß kann die Spülleitung als flexibler Spülschlauch ausgebildet sein, welcher an der Verbindungsstelle zur Punktionskanüle eine Durchmesserverengung, eine Schrumpfschlauch- oder Klebeverbindung aufweist. Zur Herstellung der Durchmesserverengung kann z.B. der Spülschlauch mit einer Heißluftpistole in einem Bereich von 1 cm bis 2 cm erhitz und gedehnt werden, bis sich das Innenlumen auf ca. 0,2 mm bis 0,3 mm verkleinert. Danach wird der Spülschlauch an dieser Stelle durchtrennt und die Punktionskanüle (Durchmesser 0,8 mm bis 1,2 mm) mit einer gewissen Kraftanwendung durch den verengten Bereich des Spülschlauchs gezwängt, wodurch eine flüssigkeitsdichte Verbindung entsteht, die den bei der Follikelspülung auftretenden Druckbelastungen stand hält.

Eine besonders einfache Herstellung des zweilumigen Bereichs ist dadurch gewährleistet, wenn die Punktionskanüle in einem proximal von der Verbindungsstelle abgewandten Bereich durch eine Einstichöffnung in das Innere des flexiblen Schlauches eintritt und durch den flexiblen Spülschlauch an der Einstichöffnung flüssigkeitsdicht abgedichtet ist. Bei der Erfindung sind somit keine komplizierten Adapter zum Einkoppeln der Schlauchleitungen in die doppellumige Nadel nötig, wie beispielsweise bei der eingangs zitierten EP 1 158 913.

Die erfindungsgemäße Punktionsvorrichtung eignet sich nicht nur für die Eizellengewinnung, sondern auch beispielsweise für die Entnahme von zellulärem Material aus der Brustdrüse oder die Hodenpunktion zur Spermiengewinnung, wobei lediglich die Abmessungen des einlumigen und des zweilumigen Bereichs dem jeweiligen Anwendungszweck entsprechend optimiert werden müssen.

Ein verbesserter Führungsadapter zur Befestigung einer Punktionsvorrichtung für die Entnahme einer Eizelle, an einem Ultraschallkopf, zeichnet sich dadurch aus, dass der Führungsadapter ein Einweg-Führungsrohr, vorzugsweise aus Kunststoff, und ein Verbindungselement samt Klemmelement, vorzugsweise aus Metall aufweist, wobei die vom Klemmelement abgewandte Seite des Verbindungselementes eine Halbschale zum Einklipsen des Einweg-Führungsrohres aufweist.

Nachdem das autoklavierbare Verbindungselement mit dem Klemmelement auf den Vaginal-Ultraschallkopf geklipst ist, wird über den Ultraschallkopf und das Verbindungselement eine dünne Schutzhülle (steriles Kondom) gestülpt. Danach kann das sterile Einweg-Führungsrohr in die Halbschale des Verbindungselements eingeklipst werden.

Der Vorteil des erfindungsgemäßen Führungsadapters liegt vor allem darin, dass alle unmittelbar für die Follikelpunktion in der IVF benötigten Utensilien außerhalb der Schutzhülle des Ultraschallkopfes liegen und Einwegprodukte sind, während bisherige System zwar Einweg-Punktionsnadeln aufweisen, die jedoch in Kontakt mit Führungsvorrichtungen stehen, die sterilisiert werden müssen.

Die Erfindung wird im Folgenden anhand von Zeichnungen näher erläutert. Es zeigen:
- Fig. 1: eine erfindungsgemäße doppellumige Punktionsvorrichtung für die Entnahme einer organischen Probe, insbesondere einer Eizelle, teilweise in einer Schnittdarstellung;
- Fig. 2: Detail X in Fig. 1 in einer vergrößerten Schnittdarstellung;
- Fig. 3: Detail Y in Fig. 1 in einer vergrößerten Schnittdarstellung;
- Fig. 4: einen Führungsadapter der Punktionsvorrichtung gemäß Fig. 1 in einer Draufsicht;
- Fig. 5 und Fig. 6: Schnittdarstellungen des Führungsadapters gemäß Linie V-V bzw. VI-VI in Fig. 4;
- Fig. 7: eine bevorzugte Variante eines zweiteiligen Führungsadapters, befestigt auf einem Ultraschallkopf; sowie die
- Fig. 8 und Fig. 9: vergrößerte Details des Führungsadapters gemäß Fig. 7.

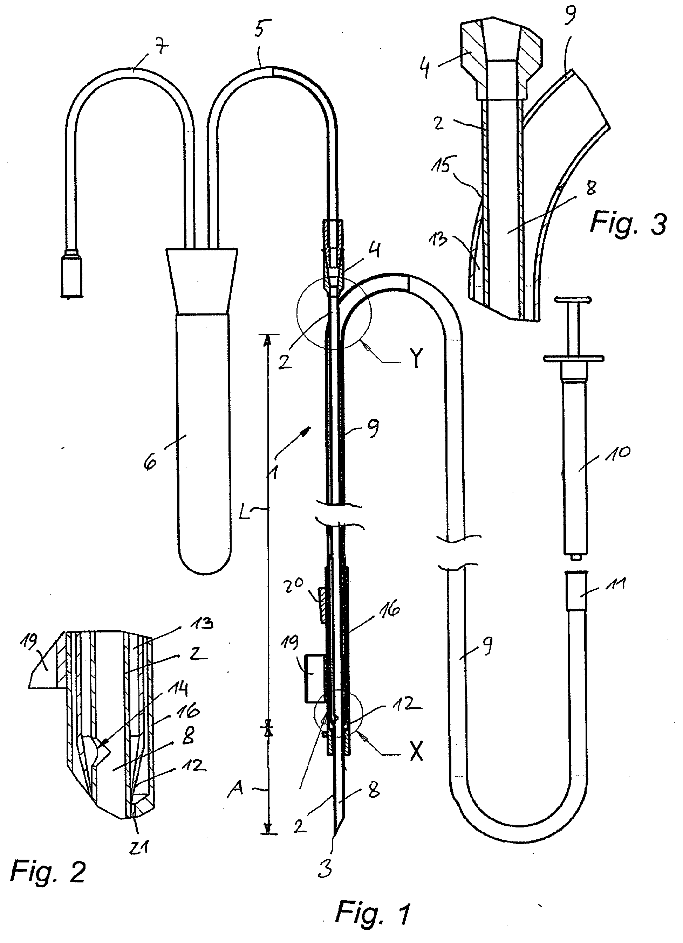

Die Punktionsvorrichtung 1 gemäß Fig. 1 bis Fig. 3 für die Entnahme einer organischen Probe weist eine Punktionskanüle 2 mit einer geschliffenen Nadelspitze 3 am distalen Ende auf, wobei an deren proximalen Ende 4 über einen Verbindungsschlauch 5 ein Aufnahmebehälter 6 für die organische Probe, beispielsweise eine Eizelle, angeschlossen werden kann. Der Aufnahmebehälter weist eine Anschlussleitung 7 zu einer hier nicht weiter dargestellten Vakuumquelle auf, um im Lumen der als Saugkanal 8 ausgeführten Punktionskanüle 2 den für die Probengewinnung nötigen Unterdruck erzeugen zu können.

Weiters ist eine als flexibler Spülschlauch ausgeführte Spülleitung 9 vorgesehen, mit welcher ein Spülmittel aus einem am proximalen Ende 11 befestigen Spülmittelbehälter 10 eingebracht werden kann. Der Spülmittelbehälter 10 kann beispielsweise als Kolbenspritze ausgeführt sein, welche über einen Luer-Anschluss am proximalen Ende 11 angesteckt werden kann. Durch eine gleichzeitige, aufeinander abgestimmte Saug- und Spültätigkeit kann ein optimales Ergebnis bei der Follikelspülung erzielt werden. Die Spülleitung 9 umfasst die Punktionskanüle 2 und ist an einer von der Nadelspitze 3 ca. 6 cm bis 9 cm beabstandeten Verbindungsstelle 12 flüssigkeitsdicht an dieser befestigt. Es entsteht so zwischen der Außenwand der Punktionskanüle 2 und der Innenwand der Spülleitung 9 ein Spülkanal 13, welcher an dessen distalem Ende zumindest eine Übertrittsöffnung 14 in den Saugkanal 8 aufweist (siehe Fig. 2). Durch die erfindungsgemäße Ausführung besteht im Anschluss an die Nadelspitze ein dünner, einlumiger Punktionsbereich A, welcher sich hervorragend für die Eizellengewinnung eignet.

Die Spülleitung 9 besteht beispielsweise aus einem flexiblen Schlauch, welcher an der flüssigkeitsdichten Verbindungsstelle 12 eine Durchmesserverengung, eine Schrumpfschlauchverbindung oder eine Klebeverbindung aufweist.

Wie im Detail in Fig. 3 dargestellt, tritt die Punktionskanüle 2 in einem proximal von der Verbindungsstelle 12 abgewandeten Bereich durch eine Einstichöffnung 15 in das Innere des flexiblen Schlauchs 9 ein, wobei die Einstichöffnung 15 durch den flexiblen Schlauch flüssigkeitsdicht abgedichtet wird.

Der einlumige Punktionsbereich A anschließend an die Nadelspitze 3 beträgt je nach Anwendungsgebiet beispielsweise 10% bis 25% der Länge L des doppellumigen Bereichs der Punktionskanüle 2, wobei der Außendurchmesser der Punktionskanüle 2 beispielsweise 0,8 mm bis 1,2 mm beträgt.

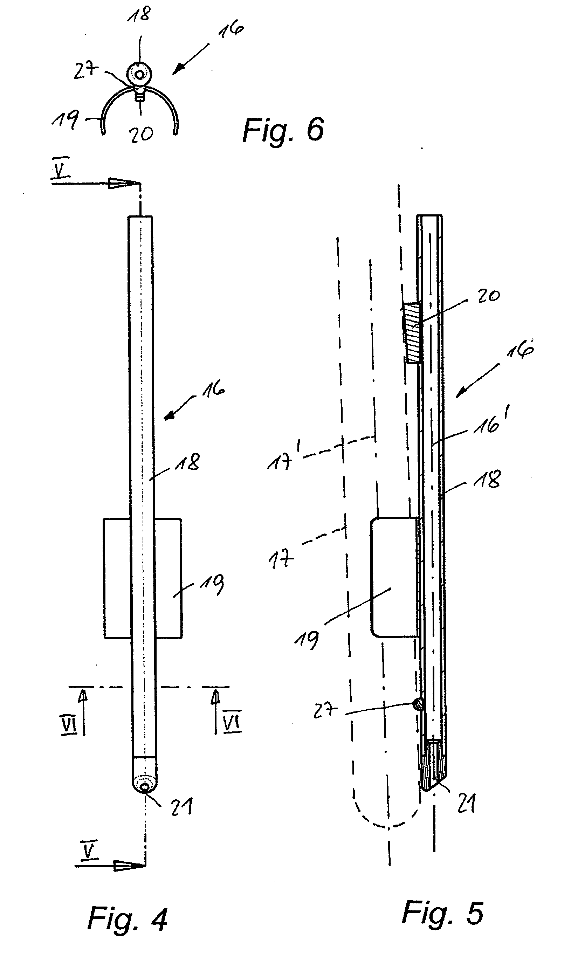

Die Punktionsvorrichtung 1 weist im doppellumigen Bereich einen Führungsadapter 16 für einen Ultraschallkopf 17 auf, der auf die Spülleitung bzw. auf den flexiblen Spülschlauch 9 aufsteckbar ist, bis zur Verbindungsstelle 12 reicht und den einlumigen Punktionsbereich A frei lässt. Wie im Detail einer ersten Ausführungsvariante in den Fig. 4 bis Fig. 6 dargestellt, besteht der Führungsadapter 16 im Wesentlichen aus einer rohrförmigen Aufnahme 18 mit einem Klemmelement 19 zur Befestigung des Führungsadapters 16 am strichliert dargestellten Ultraschallkopf 17. Am distalen Ende weist der Adapter 16 eine Öffnung 21 auf, durch die die Punktionskanüle austritt und zentriert wird.

Weiters weist der Führungsadapter 16 eine keilförmige Auflage 20 auf, mit welcher ein Winkel von ca. 3° bis 10° zwischen den Längsachse 16' des Führungsadapters 16 und der Längsachse 17' des Ultraschallkopfes 17 einstellbar ist.

An der Unterseite des Führungsadapters 16 befindet sich ein Rastelement 27, das in eine Bohrung im Sensorkopf 17 einrastet und den Führungsadapter 16 in einer vorgegebenen Position fixiert. Die Punktionsvorrichtung 1 wird erst in situ im Führungsadapter 16 vorgeschoben, sodass der Punktionsbereich A frei liegt.

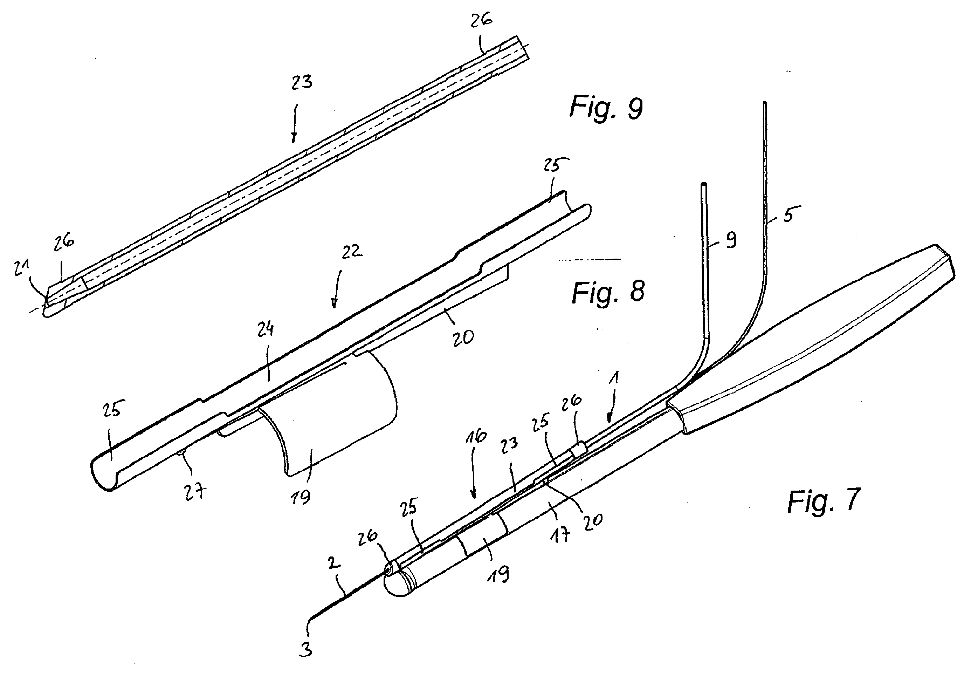

Gemäß einer zweiten, in den Fig. 7 bis Fig. 9 dargestellten Ausführungsvariante, ist der Führungsadapter 16 zur Befestigung einer Punktionsvorrichtung 1 an einem Ultraschallkopf 17, zweistückig ausgebildet und besteht aus einem Verbindungselement 22, vorzugsweise aus Metall, und einem Einweg-Führungsrohr 23, vorzugsweise aus Kunststoff. Die einzelnen Teile des Führungsadapters 16 sind im vergrößerten Maßstab in den Fig. 8 (metallisches Verbindungselement 22) und Fig. 9 (Längsschnitt des Einweg-Führungsrohrs 23) dargestellt.

Das Verbindungselement 22 weist ein Klemmelement 19 zur Befestigung am Ultraschallkopf 17 auf, wobei auf der vom Klemmelement 19 abgewandten Seite eine Halbschale 24 zum Einklipsen des Einweg-Führungsrohrs 23 ausgebildet ist. Dem Ultraschallkopf 17 mit dem daran befestigten Verbindungselement 22 wird eine dünne, sterile Schutzhülle übergestülpt, wobei das Einweg-Führungsrohr 23 nach dem Überziehen der Schutzhülle in die Halbschale 24 des Verbindungselements 22 einklipsbar ist. Nach der Anwendung kann das Einweg-Führungsrohr 23 entsorgt und das metallische Verbindungselement 22 in herkömmlicher Weise sterilisiert werden.

Die Halbschale 24 des Verbindungselements 22 weist Endbereiche 25 auf, die jeweils an einem Absatz 26 des eingeklipsten Einweg-Führungsrohrs 23 anliegen, sodass ein rutschfester Sitz des Führungsrohrs 23 gewährleistet ist.

## Patentansprüche

1. Punktionsvorrichtung (1) für die Entnahme einer organischen Probe, insbesondere einer Eizelle für die In-Vitro-Fertilisierung, mit einer zu einer Nadelspitze (3) zulaufenden Punktionskanüle (2), deren proximales Ende (4) mit einem Aufnahmebehälter (6) für die organische Probe verbindbar ist, wobei das Lumen der Punktionskanüle (2) als Saugkanal (8) ausgebildet ist, sowie mit einer Spülleitung (9), welche zur Zufuhr eines Spülmittels dient und am proximalen Ende (11) mit einem Spülmittelbehälter (10) verbindbar ist, **dadurch gekennzeichnet, dass** im Anschluss an die Nadelspitze (3) ein einlumiger Punktionsbereich (A) ausgebildet ist, an welchen ausgehend von einer Verbindungsstelle (12), in welcher die Spülleitung (9) flüssigkeitsdicht an der Punktionskanüle (2) befestigt ist, ein doppellumiger Bereich anschließt, in welchem die Punktionskanüle (2) in der Spülleitung (9) verläuft, so dass zwischen der Außenwand der Punktionskanüle (2) und der Innenwand der Spülleitung (9) ein Spülkanal (13) ausgebildet ist, welcher an dessen distalem Ende zumindest eine Übertrittsöffnung (14) in den Saugkanal (8) der Punktionskanüle (2) aufweist.

2. Punktionsvorrichtung nach Anspruch 1, **dadurch gekennzeichnet, dass** die Spülleitung (9) als flexibler Spülschlauch ausgebildet ist, welcher an der Verbindungsstelle (12) zur Punktionskanüle (2) eine Durchmesserverengung, eine Schrumpfschlauchverbindung oder eine Klebeverbindung aufweist.

3. Punktionsvorrichtung nach Anspruch 2, **dadurch gekennzeichnet, dass** die Punktionskanüle (2) in einem proximal von der Verbindungsstelle (12) abgewandten Bereich durch eine Einstichöffnung (15) in das Innere des flexiblen Schlauches (9) eintritt und durch den flexiblen Spülschlauch an der Einstichöffnung (15) flüssigkeitsdicht abgedichtet ist.

4. Punktionsvorrichtung nach einem der Ansprüche 1 bis 3, **dadurch gekennzeichnet, dass** der einlumige Punktionsbereich (A) anschließend an die Nadelspitze (3) 10% bis 25% der Länge (L) des doppellumigen Bereichs der Punktionskanüle (2) beträgt.

5. Punktionsvorrichtung nach einem der Ansprüche 1 bis 4, **dadurch gekennzeichnet, dass** der Außendurchmesser der Punktionskanüle zumindest im einlumigen Punktionsbereich (A) anschließend an die Nadelspitze (3) 0,8 mm bis 1,2 mm beträgt.

6. Punktionsvorrichtung nach einem der Ansprüche 1 bis 5, **dadurch gekennzeichnet, dass** die Punktionsvorrichtung einen Führungsadapter (16) für einen Ultraschallkopf (17) aufweist, der im doppellumigen Bereich auf die Spülleitung (9) aufsteckbar ist, bis zur Verbindungsstelle (12) reicht und den einlumigen Punktionsbereich (A) frei lässt.

7. Punktionsvorrichtung nach Anspruch 6, **dadurch gekennzeichnet, dass** der Führungsadapter (16) im Wesentlichen aus einer rohrförmigen Aufnahme (18) und einem Klemmelement (19) zur Befestigung des Führungsadapters (16) am Ultraschallkopf (17) besteht.

8. Punktionsvorrichtung nach Anspruch 6, **dadurch gekennzeichnet, dass** der Führungsadapter (16) zweistückig ausgebildet ist und aus einem Verbindungselement (22), vorzugsweise aus Metall, und einem Einweg-Führungsrohr (23), vorzugsweise aus Kunststoff, besteht.

9. Punktionsvorrichtung nach Anspruch 8, **dadurch gekennzeichnet, dass** das Verbindungselement (22) ein Klemmelement (19) zur Befestigung des Verbindungselements (22) am Ultraschallkopf (17) und auf der vom Klemmelement (19) abgewandten Seite eine Halbschale (24) zum Einklipsen des Einweg-Führungsrohrs (23) aufweist.

10. Punktionsvorrichtung nach Anspruch 9, **dadurch gekennzeichnet, dass** die Halbschale (24) des Verbindungselements (22) Endbereiche (25) aufweist, die jeweils an einem Absatz (26) des eingeklipsten Einweg-Führungsrohrs (23) anliegen.

11. Punktionsvorrichtung nach einem der Ansprüche 7 bis 10, **dadurch gekennzeichnet, dass** der Führungsadapter (16) eine keilförmige Auflage (20) aufweist, mit welcher zwischen den Längsachse (16') des Führungsadapters (16) und der Längsachse (17') des Ultraschallkopfes (17) ein Winkel von ca. 5° bis 10° einstellbar ist.

## Claims

1. Puncturing device (1) for taking an organic sample, in particular an egg cell for in-vitro fertilization, with a puncturing cannula (2) ending in a needle tip (3), whose proximal end (4) is connectable to a receiving vessel (6) for the organic sample, the lumen of the puncturing cannula (2) being configured as a suction channel (8), and with a flush line (9) which supplies a flushing medium and is connected at its proximal end to a container (10) for the flushing medium, **characterised in that** next to the needle tip (3) a single-lumen puncturing region (A) is provided, which - departing from a joining site (12) where the flush line (9) is sealingly attached to the puncturing cannula (2) - is connected to a double-lumen region, in which the puncturing cannula (2) runs inside the flushing line (9), such that a flushing channel (13) is formed between the exterior wall of the puncturing cannula (2) and the interior wall of the flush line (9), which flushing channel (13) is provided at its distal end with at least one flow opening (14) leading into the suction channel (8) of the puncturing cannula (2).

2. Puncturing device according to claim 1, **characterised in that** the flush line (9) is configured as a flexible flushing tube, which at the joining site (12) with the puncturing cannula (2) has a reduction of diameter, a shrink-on fit or a glued connection.

3. Puncturing device according to claim 2, **characterised in that** the puncturing cannula (2) enters the interior of the flexible tube (9) via a puncture (15) at a region proximal from the joining site (12), and is tightly sealed by the flexible flushing tube at the puncture (15).

4. Puncturing device according to any of claims 1 to 3, **characterised in that** the single-lumen puncturing region (A) next to the needle tip (3) has a length which is 10% to 25% of the length (L) of the double-lumen region of the puncturing cannula (2).

5. Puncturing device according to any of claims 1 to 4, **characterised in that** the exterior diameter of the puncturing cannula amounts to between 0,8 mm and 1,2 mm, at least in the single-lumen puncturing region (A) next to the needle tip (3).

6. Puncturing device according to any of claims 1 to 5, **characterised in that** the puncturing device has a guiding adaptor (16) for an ultrasonic head (17), which can be attached to the flush line (9) in the double-lumen region, and which extends up to the joining site (12) and leaves the single-lumen puncturing region (A) uncovered.

7. Puncturing device according to claim 6, **characterised in that** the guiding adaptor (16) consists essentially of a tubular receiving element (18) and a clipping element (19) for attaching the guiding adaptor (16) to the ultrasonic head (17).

8. Puncturing device according to claim 6, **characterised in that** the guiding adaptor (16) is designed as a two-piece element consisting of a connecting element (22), preferably made of metal, and a one-way guiding tube (23), preferably made of plastic.

9. Puncturing device according to claim 8, **characterised in that** the connecting element (22) is provided with a clipping element (19) for attaching the connecting element (22) to the ultrasonic head (17), and with a semi-circular shell (24) for snapping-on the one-way guiding tube (23) on the side opposite the clipping element (19).

10. Puncturing device according to claim 9, **characterised in that** the semi-circular shell (24) of the connecting element (22) has end regions (25), either of which lies adjacent to a step (26) of the snapped-on one-way guiding tube (23).

11. Puncturing device according to any of claims 7 to 10, **characterised in that** the guiding adaptor (16) has a wedge-shaped support element (20), by means of which an angle of approximately 5° to 10° can be set between the longitudinal axis (16') of the guiding adaptor (16) and the longitudinal axis (17') of the ultrasonic head (17).

## Revendications

1. Dispositif de ponction (1) pour le prélèvement d'un échantillon organique, notamment d'une nanocellule pour la fertilisation in vitro, comportant une canule de ponction (2) rejoignant une pointe d'aiguille (3) et dont l'extrémité proximale (4) peut être reliée à un réceptacle (6) pour l'échantillon organique,
le lumen de la canule de ponction (2) étant réalisé sous la forme d'un canal d'aspiration (8) et il peut être relié à une conduite de rinçage (9) pour fournir un agent de rinçage et son extrémité proximale (11) peut être reliée à un récipient de rinçage (10),
**caractérisé en ce qu'**
la pointe d'aiguille (3), se poursuit par une zone de ponction (A) en forme de lumen simple auquel, partant d'un point de liaison (12), est fixée la conduite de rinçage (9) de manière étanche au liquide au niveau de la canule de ponction (2), puis par une zone à double lumen dans laquelle la canule de ponction (2) rejoint la conduite de rinçage (9) de façon à réaliser un canal de rinçage (13) entre la paroi extérieure de la canule de ponction (2) et la paroi intérieure de la conduite de rinçage (9), l'extrémité distale de ce canal ayant au moins un orifice de passage (14) dans le canal d'aspiration (8) de la canule de ponction (2).

2. Dispositif de ponction selon la revendication 1,
**caractérisé en ce que**
la conduite de rinçage (9) est un tuyau souple ayant au point de liaison (12) avec la canule de ponction (2), ou un rétrécissement de diamètre, ou une liaison rétractée ou une liaison collée.

3. Dispositif de ponction selon la revendication 2,
**caractérisé en ce que**
dans sa zone non tournée vers la zone proximale du point de jonction (12), la canule de ponction (2) pénètre à travers une perforation (15) à l'intérieur du tube souple (9), et le tube souple de rinçage assure l'étanchéité au liquide au niveau de cette perforation (15).

4. Dispositif de ponction selon l'une des revendications 1 à 3,
**caractérisé en ce que**
la zone de ponction (A) à lumen simple adjacente à la pointe d'aiguille (3), représente entre 10 % et 25 % de la longueur (L) de la zone à double lumen de la canule de ponction (2).

5. Dispositif de ponction selon l'une des revendications 1 à 4,
**caractérisé en ce que**
le diamètre extérieur de la canule de ponction présente une zone de ponction à lumen simple (A) adjacente à la pointe d'aiguille (3) qui correspond à 0,8 mm à 1,2 mm.

6. Dispositif de ponction selon l'une des revendications 1 à 5,
**caractérisé en ce qu'**
il comporte un adaptateur de guidage (16) pour une tête à ultrasons (17) qui peut s'engager dans la zone à double lumen sur la conduite de rinçage (9) jusqu'au point de jonction (12) et laisse libre la zone de ponction (A) à lumen simple.

7. Dispositif de ponction selon la revendication 6,
**caractérisé en ce que**
l'adaptateur de guidage (16) est composé principalement d'un logement (18) essentiellement tubulaire et d'un élément de serrage (19) pour fixer l'adaptateur de guidage (16) à la tête à ultrasons (17).

8. Dispositif de ponction selon la revendication 6,
**caractérisé en ce que**
l'adaptateur de guidage (16) est réalisé en deux parties et se compose d'un élément de liaison (22) de préférence en métal et d'un tube de guidage (23) à usage unique, de préférence en matière plastique.

9. Dispositif de ponction selon la revendication 8,
**caractérisé en ce que**
l'élément de liaison (22) comporte un élément de serrage (19) pour la fixation de l'élément de liaison (22) à la tête à ultrasons (17) et sur le côté non tourné vers l'élément de serrage (19), il est prévu une demi-coquille (24) pour clipser le tube de guidage jetable (23).

10. Dispositif de ponction selon la revendication 9,
**caractérisé en ce que**
la demi-coquille (24) de l'élément de liaison (22) comporte des zones d'extrémité (25) qui s'appliquent respectivement contre un épaulement (26) du tube de guidage jetable (23), clipsé.

11. Dispositif de ponction selon l'une des revendications 7 à 10,
**caractérisé en ce que**
l'adaptateur de guidage (16) comporte un appui (20) en forme de coin par lequel on peut former un angle d'environ 5° à 10° entre l'axe longitudinal (16') de l'adaptateur de guidage (16) et l'axe longitudinal (17') de la tête à ultrasons (17).
